# EUROPEAN PATENT SPECIFICATION

(11) **EP 4 637 586 B1**
(45) Date of publication and mention of the grant of the patent: **22.04.2026**
(21) Application number: 24703419.2
(22) Date of filing: 08.01.2024
(51) Int. Cl.: A61B 17/32, A61B 17/22, A61F 2/24

(54) **HEART VALVE TISSUE CUTTING DEVICE**
HERZKLAPPENGEWEBESCHNEIDVORRICHTUNG
DISPOSITIF DE COUPE DE TISSU DE VALVULE CARDIAQUE

(30) Priority: 19.01.2023 US 202363439904 P
(43) Date of publication of application: 29.10.2025
(73) Proprietor: Pi-Cardia Ltd., 7632805 Rehovot (IL)
(72) Inventor: SPECTOR, Ben Zion, 7032019 Be'er Yaakov (IL)
(74) Representative: Pearl Cohen UK
(86) International application number: PCT/IB2024/050154
(87) International publication number: WO 2024/154012

(56) References cited:
- CN-A- 114 041 872
- US-A1- 2020 261 107
- US-A1- 2021 346 045
- US-A1- 2022 233 210

## Description

### FIELD OF THE INVENTION

The present invention generally relates to devices for transcatheter modification of body tissue such as heart valve leaflets tissue like, e.g., mitral or aortic valve leaflets.

### BACKGROUND OF THE INVENTION

PCT Patent Application PCT/IB2020/054729 describes a transcatheter valve laceration device and method. The invention is a method and device, which can be used to perform BASILICA (Bioprosthetic or native Aortic Scallop Intentional Laceration to prevent Iatrogenic Coronary Artery obstruction), or a LAMPOON procedure (for mitral). The device is a cutting or splitting device with attention to preventing damage to neighboring tissues. The device can be implemented in other cardiologic procedures, such as tricuspidization of a bicuspid valve (turning a bicuspid valve into a tricuspid valve by cutting or splitting one of the bicuspid leaflets into two leaflets) or tricuspidization of a quadricuspid valve (lacerating one of the leaflets to turn the valve into a tricuspid valve) or splitting AML (anterior mitral leaflet) to prevent LVOTO (left ventricle outflow tract obstruction), thereby preparing the patient for safe transcatheter aortic or mitral valve replacement (TAVR/TMVR), or for other procedures that involve modifying cardiac /blood vessel tissue.

US-A1-2021/346045 describes a system configured to cut leaflet tissue at a cardiac valve comprising a guide catheter and a cutting mechanism routable through the guide catheter. The cutting mechanism includes a cutting arm and a plurality of grasping arms rotatably coupled to the cutting arm. The grasping arms are connected to the cutting arm via a central hinge and are actuatable between a closed position against the cutting arm and an open position where the grasping arms extend laterally away from the cutting arm by rotating about the hinge.

US-A1-2020/261107 describes an apparatus for excising and removing a clip or suture joining leaflets of a native heart valve. The apparatus includes an elongate shaft configured for remote access to a native heart valve, the elongate shaft having a proximal end, a distal end, and a lumen defined therein, and a clamp at the distal end of the elongate shaft, the clamp configured to receive a clip or suture joining native leaflet tissue, the clamp including a passageway extending along a length thereof in communication with the lumen. The apparatus further includes a cutter moveable within the passageway to encompass a clip or suture joining native leaflet tissue received within the clamp and cut native leaflet tissue proximate the clip or suture.

### SUMMARY

The present invention seeks to provide a device for cutting heart valve tissue, such as but not limited to, mitral or aortic valve tissue, as is described more in detail hereinbelow. For example, the invention may be used for mitral anterior leaflet laceration in a transcatheter mitral valve replacement (TMVR) procedure to prevent LVOT (left ventricle outflow tract) obstruction when the implant valve is positioned within the native valve of the patient. Methods of treatment by surgery are not claimed.

The mitral valve lies between the left atrium and the left ventricle of the heart. It has two leaflets or cusps: the anteromedial leaflet (AMVL) and the posterolateral leaflet (PMVL).

The term "cutting" refers to any kind of reduction in size or any modification in shape or form, such as but not limited to, cutting, splitting, lacerating, slicing, fracturing, chopping and the like, and the terms are used interchangeably throughout.

There is thus provided in accordance with a non-limiting embodiment of the invention a heart valve tissue cutting device including a housing formed with an elongate opening, a cutting guide arm pivoted to the housing at a distal pivot, the cutting guide arm being formed with a slot, a first actuator wire coupled to the cutting guide arm, wherein movement of the first actuator wire causes the cutting guide arm to pivot about the distal pivot, a cutting element including a blade, the cutting element being coupled to a track link which is arranged to travel along a track in the housing, and a second actuator wire coupled to the track link, wherein movement of the second actuator wire causes the track link and the cutting element to move distally or proximally along the track, and wherein when the track link is at a proximal portion of the track, the blade does not protrude out of the housing and when the track link is not at the proximal portion of the track, the blade protrudes out of the housing.

In accordance with a non-limiting embodiment of the invention the proximal portion of the track curves from a central portion of the housing towards an outer contour of the housing.

In accordance with a non-limiting embodiment of the invention the cutting guide arm, when in a closed position, fits in the elongate opening.

In accordance with a non-limiting embodiment of the invention, during distal movement of the cutting element along the track, the blade moves through the slot.

In accordance with a non-limiting embodiment of the invention the second actuator wire wraps around the distal pivot.

In accordance with a non-limiting embodiment of the invention the first actuator wire is coupled to a distal cam on the cutting guide arm, the distal cam pivoting about the distal pivot.

### BRIEF DESCRIPTION OF DRAWINGS

The present invention will be understood and appreciated more fully from the following detailed description, taken in conjunction with the drawings in which:
Fig. 1 is a simplified side view illustration of a heart valve tissue cutting device, in accordance with a non-limiting embodiment of the present invention.
Fig. 2 is a simplified top view illustration of the heart valve tissue cutting device.
Fig. 3 is simplified side view illustration of the heart valve tissue cutting device, including a cutting guide arm pivoted outwards to a first outward position, the cutting guide being formed with a slot for guiding the cutting action of a cutting element.
Fig. 4 is simplified side view illustration of the heart valve tissue cutting device, with the cutting guide arm pivoted outwards to a second outward position.
Fig. 5 is a simplified side view illustration of the heart valve tissue cutting device in the position of Fig. 4, showing a cutting element in a stowed position inside the cutting device, in which the cutting element is coupled to a track link which is arranged to travel along a track and the track link is on a proximal, curved portion of the track so that the cutting element is stowed and its blade does not protrude outwards.
Fig. 6 is a simplified illustration of the cutting element and its track link, in which the track link has moved distally from the proximal end of the curved portion of the track so that the blade of the cutting element starts to protrude outwards.
Fig. 7 is a simplified illustration of the cutting element and its track link, in which the track link has moved further distally so that the blade of the cutting element is about to enter the slot of the cutting guide arm.
Fig. 8 is a simplified illustration of the cutting element and its track link, in which the track link has moved further distally so that the blade of the cutting element now protrudes through the slot of the cutting guide arm.
Fig. 9 is a simplified illustration of the cutting element and its track link, in which the track link has moved further distally so that the cutting element has reached the end of its distal travel along the track.
Fig. 10 is a side view illustration of the opposite side of Fig. 9.
Fig. 11 is a perspective illustration of the orientation of Figs. 9 and 10.

### DETAILED DESCRIPTION

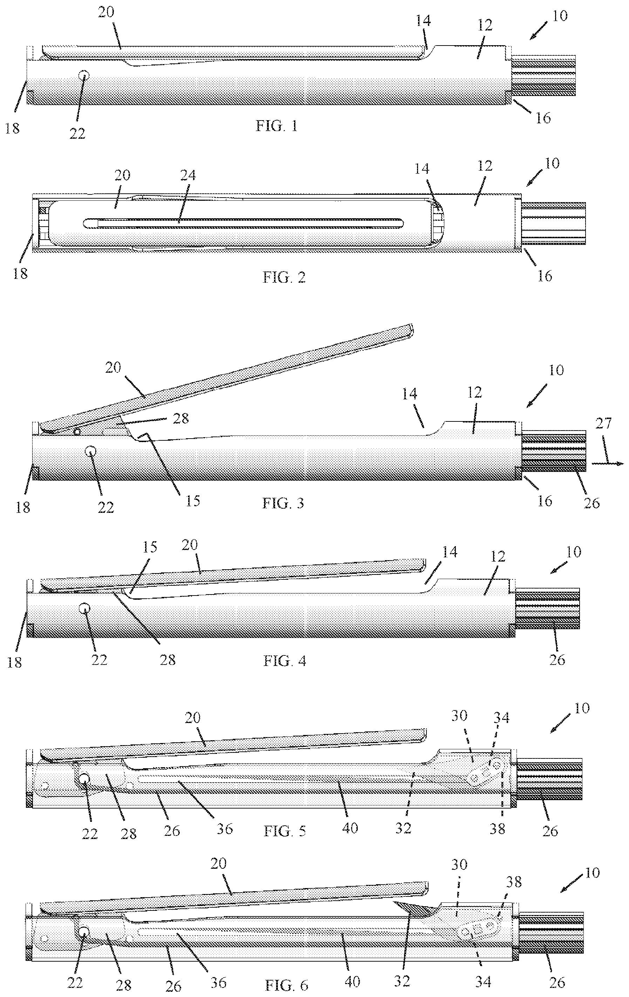

Reference is now made to Figs. 1-4, which illustrate a heart valve tissue cutting device 10, in accordance with a non-limiting embodiment of the present invention.

The heart valve tissue cutting device 10 may include a (e.g., slender, cylindrical) housing 12 formed with an elongate opening 14, which may have rounded ends. The housing 12 may be made of medical grade stainless steel or any other suitable material. A proximal end 16 of housing 12 may be open to allow passage therethrough of actuator wires (described below), which may be coupled to a manipulator (not shown) for manipulating the actuator wires. A distal end 18 of housing 12 may be blunt so as not to cause any harm to tissues. Optionally, the distal tip may be sufficiently open and/or have a guidewire channel or lumen so that a guidewire and the like can pass completely through the device from the proximal to distal ends.

As seen best in Figs. 3 and 4, elongate opening 14 may include a distal limiter 15, such as an inwardly slanted portion of opening 14 that forms a type of limiting pocket. The distal limiter 15 may be used to limit the position of the leaflet lip which is grasped by the device.

The heart valve tissue cutting device 10 may include a cutting guide arm 20 pivoted to housing 12 at a distal pivot 22. The cutting guide arm 20, when in a closed position (Figs. 1 and 2) fits in elongate opening 14. The cutting guide arm 20 may be formed with a slot 24 for guiding the cutting action of a cutting element, described hereinbelow.

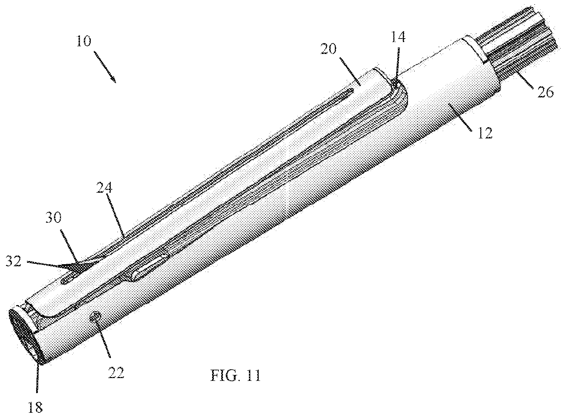

As seen in Fig. 5, a first actuator wire 26 may be coupled to a distal cam 28 on cutting guide arm 20. The actuator wire may be made of medical grade stainless steel or nitinol or polymeric wire or any other suitable material. Proximal pulling of first actuator wire 26 (in the direction of arrow 27 in Fig. 3) causes cam 28, and along with it, cutting guide arm 20 to pivot about distal pivot 22. As seen in Fig. 3, cutting guide arm 20 may be pivoted about distal pivot 22 to swing outwards to a first outward position. As seen in Fig. 4, cutting guide arm 20 may be pivoted outwards to a second outward position. One of the purposes of the two positions is described after Fig. 11 in the description of one of the methods of using the device. It will be appreciated that in use of the device, the cutting guide arm is also used as a positioning arm and as a grasping arm used to grasp or clamp cardiac tissue which is to be cut.

Reference is now made to Fig. 5. The heart valve tissue cutting device 10 may include a cutting element 30, which is here shown in a stowed position inside the cutting device. The cutting element 30 may include a blade 32. The cutting element 30 may be coupled to a track link 34, which is arranged to travel along a track 36. In the position of Fig. 5, the track link 34 is on a proximal, curved portion 38 of track 36 so that the cutting element 30 is stowed and its blade 32 does not protrude outwards. The proximal curved portion 38 of track 36 curves from a central portion of housing 12 towards an outer contour of housing 12. A second actuator wire 40 may be coupled to track link 34. As mentioned above, the first and second actuator wires may be coupled to a manipulator (not shown) for manipulating the actuator wires.

Reference is now made to Fig. 6. The track link 34 has moved distally from the proximal end of the curved portion 38 of track 36 so that blade 32 of cutting element 30 starts to protrude outwards.

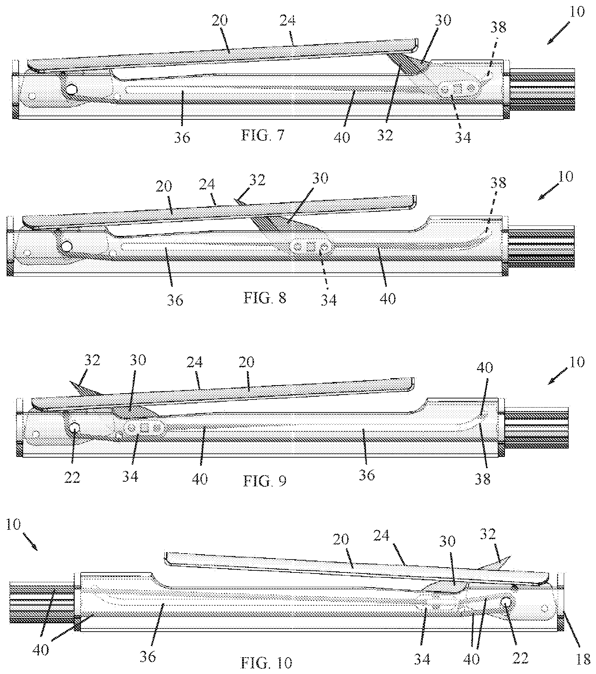

Reference is now made to Fig. 7. The track link 34 has moved further distally so that blade 32 of cutting element 30 is about to enter the slot 24 of cutting guide arm 20.

Reference is now made to Fig. 8. The track link 34 has moved further distally so that blade 32 of cutting element 30 now protrudes through slot 24 of cutting guide arm 20.

Reference is now made to Fig. 9. The track link 34 has moved further distally so that cutting element 30 has reached the end of its distal travel along track 36. Fig. 10 is a side view illustration of the opposite side of Fig. 9. It is seen in Fig. 10 that second actuator wire 40 may wrap around distal pivot 22. Alternatively, second actuator wire 40 may wrap around a different pivot.

Fig. 11 is a perspective illustration of the orientation of Figs. 9 and 10, clearly showing blade 32 of cutting element 30 protruding through slot 24 of cutting guide arm 20.

In one method of using the device, the device 10 may be percutaneously introduced through vasculature to the desired site (e.g., a leaflet which is to be cut or split or otherwise modified) over a delivery system (e.g., in which the device is initially sheathed and delivered over a guidewire and then unsheathed). The cutting guide arm 20 may be first expanded outwards to the first position of Fig. 3. In this position, the cutting guide arm 20 is used as a positioning arm that bypasses the leaflet on its ventricular side. The actuator then moves the cutting guide arm 20 to the second position of Fig. 4 so as to grasp or clamp the leaflet, while allowing the imaging system to visualize the position of the grasp. The slot 24 or the shape of the device can visually indicate to the surgeon the cut or split line of split. Once the surgeon verifies the proper location for cutting, all the while grasping the leaflet in the second position of Fig. 4, the blade 32 of cutting element 30 is actuated and moved distally to cut or split the leaflet. The distal limiter 15 of the elongate opening 14 limits the position of the leaflet lip and allows a final cutting action of blade 32 to split through the leaflet lip.

It is noted that the method splits the leaflet by grasping the leaflet and starting to cut or split from the side of the leaflet away from the lip. That is, for the anterior leaflet, the split starts from the base of the leaflet towards the lip in the direction towards the left ventricle. This method of splitting allows for better control of the puncture point (the point where the blade punctures the leaflet) and results in a smooth split since while grasping and actuating the splitter there is constant tension on the leaflet. Splitting by starting in the opposite direction could possibly crease or crimp the leaflet and compromise the quality of the split.

## Claims

1. A heart valve tissue cutting device (10) comprising:
a housing (12) formed with an elongate opening (14);
a cutting guide arm (20) pivoted to said housing (12) at a distal pivot (22), said cutting guide arm (20) being formed with a slot (24);
a first actuator wire (26) coupled to said cutting guide arm (20), wherein movement of said first actuator wire (26) causes said cutting guide arm (20) to pivot about said distal pivot (22);
a cutting element (30) comprising a blade (32), said cutting element (30) being coupled to a track link (34) which is arranged to travel along a track (36) in said housing (12); and
a second actuator wire (40) coupled to said track link (34), wherein movement of said second actuator wire (40) causes said track link (34) and said cutting element (30) to move distally or proximally along said track (36), and wherein when said track link (34) is at a proximal portion of said track (36), said blade (32) does not protrude out of said housing (12) and when said track link (34) is not at said proximal portion of said track (36), said blade (32) protrudes out of said housing (12).

2. The heart valve tissue cutting device (10) according to claim 1, wherein proximal portion of said track (36) curves from a central portion of said housing (12) towards an outer contour of said housing (12).

3. The heart valve tissue cutting device (10) according to claim 1, wherein said cutting guide arm (20), when in a closed position, fits in said elongate opening (14).

4. The heart valve tissue cutting device (10) according to claim 1, wherein during distal movement of said cutting element (30) along said track (36), said blade (32) moves through said slot (24).

5. The heart valve tissue cutting device (10) according to claim 1, wherein said second actuator wire (40) wraps around said distal pivot (22).

6. The heart valve tissue cutting device (10) according to claim 1, wherein said first actuator wire (26) is coupled to a distal cam on said cutting guide arm (20), said distal cam pivoting about said distal pivot (22).

7. The heart valve tissue cutting device (10) according to claim 1, wherein said elongate opening (14) comprises a distal, inwardly slanted portion.

## Patentansprüche

1. Schneidvorrichtung (10) für Herzklappengewebe, umfassend:
ein Gehäuse (12), das mit einer langgestreckten Öffnung (14) ausgebildet ist;
einen Schneidführungsarm (20), der an einem distalen Drehpunkt (22) schwenkbar mit dem Gehäuse (12) verbunden ist, wobei der Schneidführungsarm (20) mit einem Schlitz (24) ausgebildet ist;
einen ersten Betätigungsdraht (26), der an den Schneidführungsarm (20) gekoppelt ist, wobei Bewegung des ersten Betätigungsdrahts (26) bewirkt, dass der Schneidführungsarm (20) um den distalen Drehpunkt (22) schwenkt;
ein Schneidelement (30), das eine Klinge (32) umfasst, wobei das Schneidelement (30) an ein Schienenglied (34) gekoppelt ist, das dazu eingerichtet ist, sich entlang einer Schiene (36) in dem Gehäuse (12) voranzubewegen; und
einen zweiten Betätigungsdraht (40), der an das Schienenglied (34) gekoppelt ist, wobei Bewegung des zweiten Betätigungsdrahts (40) bewirkt, dass sich das Schienenglied (34) und das Schneidelement (30) distal oder proximal entlang der Schiene (36) bewegen, und wobei, wenn sich das Schienenglied (34) an einem proximalen Abschnitt der Schiene (36) befindet, die Klinge (32) nicht aus dem Gehäuse (12) ragt, und wenn sich das Schienenglied (34) nicht an dem proximalen Abschnitt der Schiene (36) befindet, die Klinge (32) aus dem Gehäuse (12) ragt.

2. Schneidvorrichtung (10) für Herzklappengewebe nach Anspruch 1, wobei der proximale Abschnitt der Schiene (36) von einem mittleren Abschnitt des Gehäuses (12) in Richtung eines äußeren Umrisses des Gehäuses (12) gekrümmt ist.

3. Schneidvorrichtung (10) für Herzklappengewebe nach Anspruch 1, wobei der Schneidführungsarm (20), wenn in einer geschlossenen Stellung, in die langgestreckte Öffnung (14) passt.

4. Schneidvorrichtung (10) für Herzklappengewebe nach Anspruch 1, wobei, während distaler Bewegung des Schneidelements (30) entlang der Schiene (36), sich die Klinge (32) durch den Schlitz (24) bewegt.

5. Schneidvorrichtung (10) für Herzklappengewebe nach Anspruch 1, wobei der zweite Betätigungsdraht (40) den distalen Drehpunkt (22) umschlingt.

6. Schneidvorrichtung (10) für Herzklappengewebe nach Anspruch 1, wobei der erste Betätigungsdraht (26) an einen distalen Nocken an dem Schneidführungsarm (20) gekoppelt ist, wobei der distale Nocken um den distalen Drehpunkt (22) schwenkt.

7. Schneidvorrichtung (10) für Herzklappengewebe nach Anspruch 1, wobei die langgestreckte Öffnung (14) einen distalen, nach innen abgeschrägten Abschnitt umfasst.

## Revendications

1. Dispositif de coupe de tissu de valve cardiaque (10) comprenant :
un logement (12) formé avec une ouverture allongée (14) ;
un bras de guide de coupe (20) relié de manière pivotante audit logement (12) au niveau d'un pivot distal (22), ledit bras de guide de coupe (20) étant formé avec une fente (24) ;
un premier fil d'actionneur (26) couplé audit bras de guide de coupe (20), dans lequel le mouvement dudit premier fil d'actionneur (26) amène ledit bras de guide de coupe (20) à pivoter autour dudit pivot distal (22) ;
un élément de coupe (30) comprenant une lame (32), ledit élément de coupe (30) étant couplé à une liaison de voie (34) qui est agencée pour se déplacer le long d'une voie (36) dans ledit logement (12) ; et
un deuxième fil d'actionneur (40) couplé à ladite liaison de voie (34), dans lequel le mouvement dudit deuxième fil d'actionneur (40) amène ladite liaison de voie (34) et ledit élément de coupe (30) à se déplacer de manière distale ou proximale le long de ladite voie (36), et dans lequel lorsque ladite liaison de voie (34) se trouve au niveau d'une partie proximale de ladite voie (36), ladite lame (32) ne fait pas saillie hors dudit logement (12) et lorsque ladite liaison de voie (34) ne se trouve pas au niveau de ladite partie proximale de ladite voie (36), ladite lame (32) fait saillie hors dudit logement (12).

2. Dispositif de coupe de tissu de valve cardiaque (10) selon la revendication 1, dans lequel la partie proximale de ladite voie (36) se courbe depuis une partie centrale dudit logement (12) vers un contour externe dudit logement (12).

3. Dispositif de coupe de tissu de valve cardiaque (10) selon la revendication 1, dans lequel ledit bras de guide de coupe (20), lorsqu'il est dans une position fermée, s'ajuste dans ladite ouverture allongée (14).

4. Dispositif de coupe de tissu de valve cardiaque (10) selon la revendication 1, dans lequel pendant le mouvement distal dudit élément de coupe (30) le long de ladite voie (36), ladite lame (32) se déplace à travers ladite fente (24).

5. Dispositif de coupe de tissu de valve cardiaque (10) selon la revendication 1, dans lequel ledit deuxième fil d'actionneur (40) s'enroule autour dudit pivot distal (22).

6. Dispositif de coupe de tissu de valve cardiaque (10) selon la revendication 1, dans lequel ledit premier fil d'actionneur (26) est couplé à une came distale sur ledit bras de guide de coupe (20), ladite came distale pivotant autour dudit pivot distal (22).

7. Dispositif de coupe de tissu de valve cardiaque (10) selon la revendication 1, dans lequel ladite ouverture allongée (14) comprend une partie inclinée vers l'intérieur, distale.
